# EUROPEAN PATENT APPLICATION

(11) **EP 4 847 055 A1**
(43) Date of publication of application: **02.12.2026**
(21) Application number: 25824606.5
(22) Date of filing: 16.04.2025
(51) Int. Cl.: A61M 1/06

(54) **BREAST PUMP AND ASSEMBLY METHOD AND DISASSEMBLY METHOD FOR BREAST PUMP**

(30) Priority: 26.06.2024 CN 202410841076
(71) Applicant: Shenzhen Lute Jiacheng Supply Chain Management Co., Ltd., Shenzhen, Guangdong 518000 (CN)
(72) Inventor: ZHENG, Jieqing, Shenzhen, Guangdong 518000 (CN)
(74) Representative: Winter, Brandl - Partnerschaft mbB
(86) International application number: PCT/CN2025/089228
(87) International publication number: WO 2026/001216

(57) **Abstract**

Disclosed in the present application is a breast pump, including a milk storage container, a breast shield, a main unit, and a disassembly restricting member. A storage space for storing milk is formed in the milk storage container. The breast shield is detachably connected to the milk storage container, and a milk expression channel is formed in the breast shield. The main unit is detachably connected to the milk storage container and configured to generate a negative pressure for milk expression in the milk expression channel to drive the milk in the milk expression channel to flow into the storage space. The disassembly restricting member is connected to the main unit, the breast shield, or the milk storage container, and is configured to restrict disassembly between the breast shield and the milk storage container in a state where the main unit is connected to the milk storage container, and allow the disassembly between the breast shield and the milk storage container in a state where the main unit is disconnected from the milk storage container. In this way, the present application can prevent the main unit from being contaminated by the milk and producing odor, or avoid damage to electronic components in the main unit or damage to a negative pressure pump, thereby prolonging the service life of the breast pump.

## Description

### RELATED APPLICATIONS

The present application claims priority to Chinese Patent Application No. 202410841076.0, filed on June 26, 2024, which is incorporated herein by reference in its entirety.

### FIELD OF TECHNOLOGY

Embodiments of the present application relate to the technical field of maternal and infant devices, and particularly to a breast pump, and an assembly method and a disassembly method therefor.

### BACKGROUND

As an important device for assisting breastfeeding, breast pumps are widely used in modern households and medical institutions. Their convenience and practicality have been recognized by a large number of users, especially working mothers and infants with special care needs.

Although existing breast pumps are quite mature in design, there still exist some technical problems to be urgently solved in actual use. Of particular concern is that some breast pumps may experience milk backflow into the main unit during operation. This backflow issue may not only cause internal contamination of the main unit, increasing the difficulty of cleaning and maintenance and shortening the service life of the device, but also lead to hygiene and safety hazards, affecting breast milk quality and infant health. The backflow can be attributed to multiple factors, including insufficient sealing at the joint between the main unit and the milk storage container, and reverse milk flow induced by negative pressure fluctuations or sudden interruptions during milk expression.

Existing breast pump designs usually focus on optimizing the efficiency of negative pressure generation and the comfort of milk expression, while dedicated protective measures to prevent milk backflow into the main unit are relatively lacking. Common connection structures, such as simple clips and screw-on or plug-in interfaces, are easy to assemble and disassemble, but may have limitations in addressing sealing challenges under negative-pressure environments. In addition, although some breast pumps on the market have attempted to use various sealing elements or improved connection methods to reduce the risk of backflow, it is still difficult to completely avoid backflow in actual use, especially in cases of improper user operation, device aging, or untimely maintenance.

### SUMMARY

**In** view of the deficiencies in the prior art, the present application provides a breast pump, and an assembly method and a disassembly method therefor, which can solve the problem of milk backflow into the main unit due to improper disassembly.

To solve the above technical problem, one technical solution adopted in the present application is to provide a breast pump, including: a milk storage container, where a storage space for storing milk is formed in the milk storage container; a breast shield, where the breast shield is detachably connected to the milk storage container, and a milk expression channel is formed in the breast shield; a main unit, where the main unit is detachably connected to the milk storage container and configured to generate a negative pressure for milk expression in the milk expression channel to drive the milk in the milk expression channel to flow into the storage space; and a disassembly restricting member, where the disassembly restricting member is connected to the main unit, the breast shield, or the milk storage container, and is configured to restrict disassembly between the breast shield and the milk storage container in a state where the main unit is connected to the milk storage container, and allow the disassembly between the breast shield and the milk storage container in a state where the main unit is disconnected from the milk storage container.

The disassembly restricting member is connected to the breast shield and is capable of extending between the milk storage container and the main unit, and the disassembly restricting member is clamped between the main unit and the milk storage container in the state where the main unit is connected to the milk storage container.

The disassembly restricting member is made of a soft material.

The breast shield is at least partially made of a soft material.

The disassembly restricting member and the breast shield are integrally formed.

The milk storage container and the main unit define an accommodating space, and the disassembly restricting member is accommodated in the accommodating space in the state where the main unit is connected to the milk storage container.

A size of a lower portion of the accommodating space is greater than a size of an upper portion of the accommodating space, and the disassembly restricting member matches the accommodating space in shape.

The accommodating space includes an accommodating groove formed in a surface of the milk storage container close to the main unit.

The milk storage container and the breast shield define the storage space.

The milk storage container is provided with a milk pouring spout, and the disassembly restricting member is further configured to cover and seal the milk pouring spout.

To solve the above technical problem, another technical solution adopted in the present disclosure is to provide an assembly method for a breast pump, including: providing a main unit, a breast shield, a milk storage container, and a disassembly restricting member; connecting the breast shield to the milk storage container, and placing the disassembly restricting member at a preset position; and connecting the milk storage container to the main unit, such that the disassembly restricting member restricts disassembly between the breast shield and the milk storage container.

The disassembly restricting member is connected to the breast shield. The connecting the breast shield to the milk storage container, and placing the disassembly restricting member at a preset position includes: connecting the breast shield to the milk storage container, and placing the disassembly restricting member between the milk storage container and the main unit. The connecting the milk storage container to the main unit, such that the disassembly restricting member restricts disassembly between the breast shield and the milk storage container includes: connecting the milk storage container to the main unit, such that the disassembly restricting member is clamped between the main unit and the milk storage container.

To solve the above technical problem, yet another technical solution adopted in the present application is to provide a disassembly method for a breast pump, including: disassembling a milk storage container and a main unit to release restriction of a disassembly restricting member on disassembly between the milk storage container and a breast shield; and disassembling the milk storage container and the breast shield.

According to an embodiment of the present application, a breast pump includes a milk storage container, a breast shield, a main unit, and a disassembly restricting member. A storage space for storing milk is formed in the milk storage container. The breast shield is detachably connected to the milk storage container, and a milk expression channel is formed in the breast shield. The main unit is detachably connected to the milk storage container and configured to generate a negative pressure for milk expression in the milk expression channel to drive the milk in the milk expression channel to flow into the storage space. The disassembly restricting member is connected to the main unit, the breast shield, or the milk storage container, and is configured to restrict disassembly between the breast shield and the milk storage container in a state where the main unit is connected to the milk storage container, and allow the disassembly between the breast shield and the milk storage container in a state where the main unit is disconnected from the milk storage container. Thus, the problem of milk backflow into the main unit due to the fact that the main unit remains connected to the milk storage container after the breast shield and the milk storage container are disassembled by a user can be avoided, and the main unit can be prevented from being contaminated by the milk and producing odor, or the damage to electronic components in the main unit or the damage to a negative pressure pump can be avoided, thereby prolonging the service life of the breast pump.

### BRIEF DESCRIPTION OF THE DRAWINGS

To more clearly illustrate the technical solutions in the embodiments of the present disclosure or in the prior art, the accompanying drawings that need to be used in the description of the embodiments or the prior art will be briefly introduced below. Apparently, the accompanying drawings in the description below merely illustrate some embodiments of the present disclosure. Those of ordinary skill in the art may also derive other accompanying drawings from these accompanying drawings without creative efforts.
FIG. 1 is a schematic structural diagram of a breast pump according to an embodiment of the present application;
FIG. 2 is a schematic exploded view of a breast pump according to an embodiment of the present application;
FIG. 3 is a schematic cross-sectional view of a breast pump according to an embodiment of the present application;
FIG. 4 is a schematic flowchart of an assembly method for a breast pump according to one embodiment of the present application; and
FIG. 5 is a schematic flowchart of a disassembly method for a breast pump according to one embodiment of the present application.

The objective achievement, functional characteristics, and advantages of the present disclosure will be further described with reference to the accompanying drawings in conjunction with the embodiments.

### DESCRIPTION OF THE EMBODIMENTS

In the present disclosure, the terms "disposed," "provided with," and "connected" should be understood in a broad sense. For example, it may be a fixed connection, a detachable connection, or an integral connection; may be a mechanical connection or an electrical connection; and may be a direct connection, an indirect connection via an intermediate medium, or internal communication between two devices, elements or components. Those of ordinary skill in the art can understand specific meanings of the above terms in the present disclosure according to specific circumstances.

The orientations or positional relationships indicated by the terms such as "center," "longitudinal," "transverse," "length," "width," "thickness," "upper," "lower," "front," "rear," "left," "right," "vertical," "horizontal," "top," "bottom," "inner," "outer," "radial," and "circumferential" are based on the orientations or positional relationships shown in the drawings, which are merely for the convenience of describing the present application and simplifying the description, rather than indicating or implying that the device or element referred to must have a particular orientation or be configured and operated in a particular orientation, and thus should not be construed as limitations on the present application.

In addition, the terms "first" and "second" are only for descriptive purposes, and cannot be construed as indicating or implying relative importance or implying the number of technical features indicated. Thus, features defined with "first" and "second" can explicitly or implicitly include at least one of the features. In the description of the present application, "a plurality of" means at least two, for example, two, three, etc., unless otherwise specifically defined.

Furthermore, some of the above terms may be used to express other meanings in addition to the orientations or positional relationships. For example, the term "upper" may also be used to express a specific attachment or connection relationship in some cases. Those of ordinary skill in the art can understand specific meanings of the above terms in the present application according to specific circumstances.

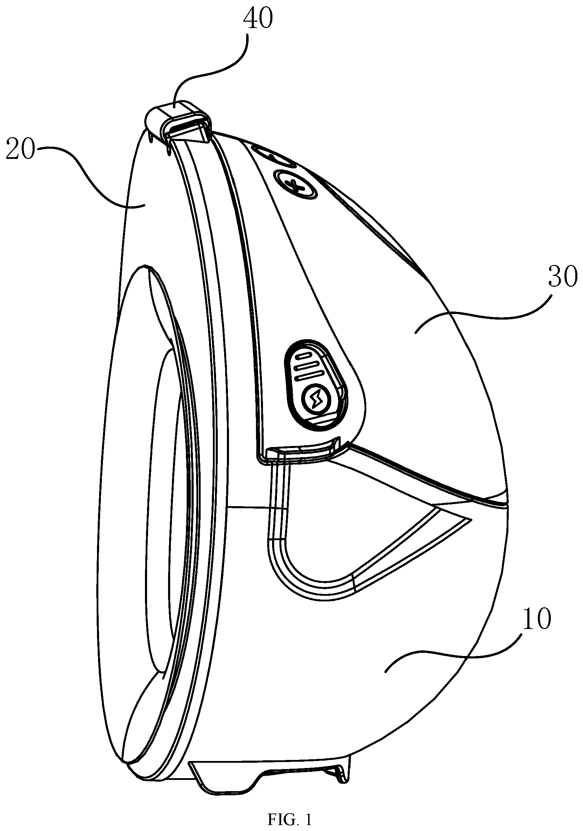

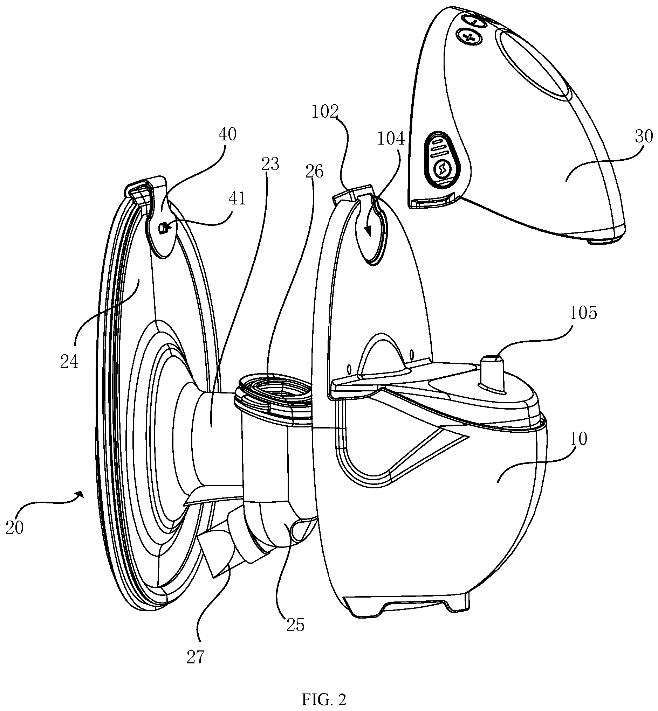

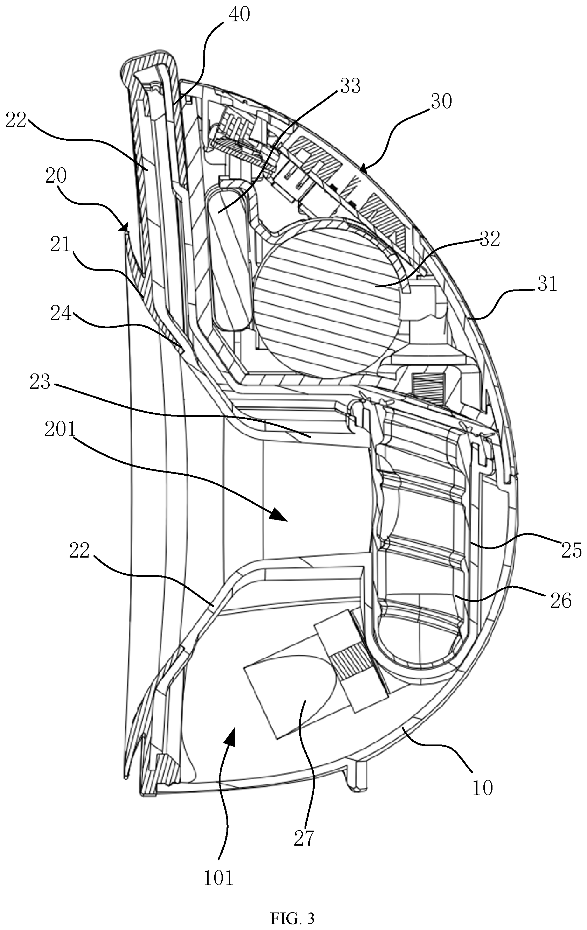

Reference is made to FIGS. 1 to 3. FIG. 1 is a schematic structural diagram of a breast pump according to an embodiment of the present application; FIG. 2 is a schematic exploded view of a breast pump according to an embodiment of the present application; and FIG. 3 is a schematic cross-sectional view of a breast pump according to an embodiment of the present application.

In this embodiment, the breast pump includes a milk storage container 10, a breast shield 20, a main unit 30, and a disassembly restricting member 40.

A storage space 101 for storing milk is formed in the milk storage container 10.

The breast shield 20 is detachably connected to the milk storage container 10, and a milk expression channel 201 is formed in the breast shield 20.

The main unit 30 is detachably connected to the milk storage container 10 and configured to generate a negative pressure for milk expression in the milk expression channel 201 to drive the milk in the milk expression channel 201 to flow into the storage space 101.

The disassembly restricting member 40 is connected to the main unit 30, the breast shield 20, or the milk storage container 10, and is configured to restrict disassembly between the breast shield 20 and the milk storage container 10 in a state where the main unit 30 is connected to the milk storage container 10, and allow the disassembly between the breast shield 20 and the milk storage container 10 in a state where the main unit 30 is disconnected from the milk storage container 10.

In this way, the problem of milk backflow into the main unit 30 due to the fact that the main unit 30 remains connected to the milk storage container 10 after the breast shield 20 and the milk storage container 10 are disassembled by a user can be avoided, and the main unit 30 can be prevented from being contaminated by the milk and producing odor, or the damage to electronic components in the main unit 30 or the damage to a negative pressure pump can be avoided, thereby prolonging the service life of the breast pump.

In one embodiment, the disassembly restricting member 40 is connected to the breast shield 20 and can extend between the milk storage container 10 and the main unit 30, and the disassembly restricting member 40 is clamped between the main unit 30 and the milk storage container 10 in the state where the main unit 30 is connected to the milk storage container 10.

It should be understood that in other embodiments, the disassembly restricting member 40 may be connected to the milk storage container 10 or to the main unit 30, as long as the disassembly restricting member 40 can restrict the disassembly between the breast shield 20 and the milk storage container 10 in the state where the main unit 30 is connected to the milk storage container 10, and allow the disassembly between the breast shield 20 and the milk storage container 10 in the state where the main unit 30 is disconnected from the milk storage container 10.

In one embodiment, the disassembly restricting member 40 is made of a soft material, such as silicone and rubber. The disassembly restricting member 40 made of the soft material can be better clamped between the main unit 30 and the milk storage container 10. In other embodiments, the disassembly restricting member 40 may also be made of a rigid material, which is not limited in the embodiments of the present application.

In one embodiment, the breast shield 20 is at least partially made of a soft material. Specifically, the breast shield 20 includes a soft rubber layer 21 made of soft rubber and a rigid rubber layer 22 made of rigid rubber, where the soft rubber layer 21 is located on the side close to the human breast. In other embodiments, the breast shield 20 may be entirely made of soft rubber or entirely made of rigid rubber, which is not limited in the embodiments of the present application. In some embodiments, a nipple tunnel portion 23 of the breast shield 20 is entirely made of rigid rubber, and a flared breast flange portion 24 has a double-layer structure of soft rubber and rigid rubber.

In one embodiment, the disassembly restricting member 40 and the breast shield 20 are integrally formed. For example, the disassembly restricting member 40 and the soft rubber layer 21 of the breast shield 20 are integrally injection-molded.

In one embodiment, the milk storage container 10 and the main unit 30 define an accommodating space, and the disassembly restricting member 40 is accommodated in the accommodating space in the state where the main unit 30 is connected to the milk storage container 10. For example, the milk storage container 10 or the main unit 30 is provided with an accommodating groove, and the accommodating space is defined after the milk storage container 10 and the main unit 30 are assembled.

In one embodiment, a size of a lower portion of the accommodating space is greater than a size of an upper portion of the accommodating space, and the disassembly restricting member 40 matches the accommodating space in shape. By setting the size of the lower portion of the accommodating space to be greater than the size of the upper portion, the disassembly restricting member 40 can be restricted from being pulled out upward in a state where the main unit 30 and the milk storage container 10 are assembled.

In one embodiment, the accommodating space includes an accommodating groove 104 formed in a surface of the milk storage container 10 close to the main unit 30. It should be understood that an accommodating groove may be formed in a surface of the main unit 30 close to the milk storage container 10, or accommodating grooves may be formed in both the main unit 30 and the milk storage container 10, as long as the accommodating space for accommodating the disassembly restricting member 40 can be defined.

In one embodiment, the milk storage container 10 and the breast shield 20 define the storage space 101. In this embodiment, the milk storage container 10 is a milk bowl, and the milk bowl and the breast shield 20 jointly define the storage space 101. In other embodiments, the milk storage container 10 may be a feeding bottle, which is not limited in the embodiments of the present application.

In one embodiment, the milk storage container 10 is provided with a milk pouring spout 102, and the disassembly restricting member 40 is further configured to cover and seal the milk pouring spout 102. Optionally, the disassembly restricting member 40 and the breast shield 20 are integrally connected at an outer edge of the breast flange portion 24. The disassembly restricting member 40 extends through the milk pouring spout 102. After the breast shield 20 and the milk bowl 10 are assembled, the disassembly restricting member 40 is placed into the accommodating groove 104, and the main unit 30 and the breast shield 20 are assembled, the disassembly restricting member 40 seals the milk pouring spout 102.

In one embodiment, the disassembly restricting member 40 is provided with a through hole 41 extending through the thickness thereof, and the through hole 41 is configured to hang the breast shield 20 on a hook for air-drying, sun-drying, etc.

In one embodiment, the main unit 30 includes a housing 31, and a negative pressure pump 32, a solenoid valve, a battery 33, a circuit board, and the like located in the housing 31.

In one embodiment, the milk storage container 10 is provided with an air tube connector 105 connected to the negative pressure pump 32 in the main unit 30.

In one embodiment, a negative pressure chamber 25 is further disposed at an end of the nipple tunnel portion 23, an internal space at the end of the nipple tunnel portion 23 is in communication with the negative pressure chamber 25, a diaphragm 26 is disposed in the negative pressure chamber 25, and the diaphragm 26 is configured for gas-liquid separation between the internal space of the nipple tunnel portion 23 and the main unit 30. After the breast shield 20 and the milk storage container 10 are assembled, a seal is formed among the negative pressure chamber 25, the diaphragm 26, and an inner wall of the milk storage container 10.

In one embodiment, a milk outlet is provided at the bottom of the negative pressure chamber 25, a check valve 27 is disposed at the milk outlet, and the check valve 27 is configured to allow the milk expressed from the milk expression channel 203 into the negative pressure chamber 25 to flow unidirectionally into the storage space 101. The check valve may be a duckbill valve. Certainly, in other embodiments, a flap-type check valve may also be used, which is not limited in the embodiments of the present application.

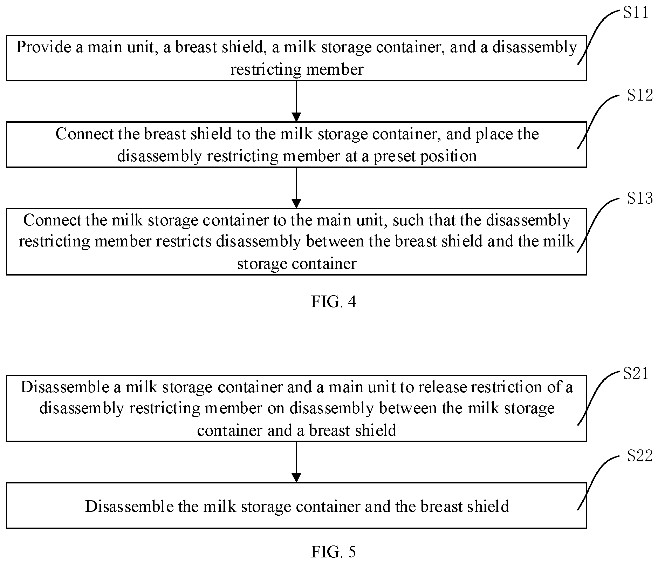

Reference is made to FIG. 4. FIG. 4 is a schematic flowchart of an assembly method for a breast pump according to one embodiment of the present application. In this embodiment, the assembly method for the breast pump includes:
Step S11: providing a main unit, a breast shield, a milk storage container, and a disassembly restricting member,
   where referring to FIGS. 1 to 3, the assembly method for the breast pump according to the embodiment of the present application can be applied to the breast pump in the above embodiment, and the milk storage container 10, the breast shield 20, the main unit 30, and the disassembly restricting member 40 are provided;
Step S12: connecting the breast shield to the milk storage container, and placing the disassembly restricting member at a preset position,
   where the preset position may be on a surface of the milk storage container 10 close to the main unit 30, or in an accommodating groove in the surface of the milk storage container 10 close to the main unit 30; and
Step S13: connecting the milk storage container to the main unit, such that the disassembly restricting member restricts disassembly between the breast shield and the milk storage container.

In one embodiment, the disassembly restricting member is connected to the breast shield.

The connecting the breast shield to the milk storage container, and placing the disassembly restricting member at a preset position includes: connecting the breast shield to the milk storage container, and placing the disassembly restricting member between the milk storage container and the main unit.

The connecting the milk storage container to the main unit, such that the disassembly restricting member restricts disassembly between the breast shield and the milk storage container includes: connecting the milk storage container to the main unit, such that the disassembly restricting member is clamped between the main unit and the milk storage container.

For example, the milk storage container 10 is connected to the main unit 30, and the disassembly restricting member 40 is clamped between the milk storage container 10 and the main unit 30. The disassembly restricting member 40 is fixedly connected to the breast shield 20, so the disassembly of the breast shield 20 is restricted. Only after the main unit 30 and the milk storage container 10 are disassembled, the breast shield 20 can be separated from the milk storage container 10.

Reference is made to FIG. 5. FIG. 5 is a schematic flowchart of a disassembly method for a breast pump according to one embodiment of the present application.

In this embodiment, the disassembly method for the breast pump includes:
Step S21: disassembling a milk storage container and a main unit to release restriction of a disassembly restricting member on disassembly between the milk storage container and a breast shield; and
Step S22: disassembling the milk storage container and the breast shield.

According to an embodiment of the present application, a breast pump includes a milk storage container, a breast shield, a main unit, and a disassembly restricting member. A storage space for storing milk is formed in the milk storage container. The breast shield is detachably connected to the milk storage container, and a milk expression channel is formed in the breast shield. The main unit is detachably connected to the milk storage container and configured to generate a negative pressure for milk expression in the milk expression channel to drive the milk in the milk expression channel to flow into the storage space. The disassembly restricting member is connected to the main unit, the breast shield, or the milk storage container, and is configured to restrict disassembly between the breast shield and the milk storage container in a state where the main unit is connected to the milk storage container, and allow the disassembly between the breast shield and the milk storage container in a state where the main unit is disconnected from the milk storage container. Thus, the problem of milk backflow into the main unit due to the fact that the main unit remains connected to the milk storage container after the breast shield and the milk storage container are disassembled by a user can be avoided, and the main unit can be prevented from being contaminated by the milk and producing odor, or the damage to electronic components in the main unit or the damage to a negative pressure pump can be avoided, thereby prolonging the service life of the breast pump.

To make the objectives, technical solutions, and advantages of the present application clearer, the present application is further described in detail below with reference to the accompanying drawings and the embodiments. It should be understood that the specific embodiments described herein are merely used to explain the present application, but not to limit the present application.

The above descriptions are only the specific embodiments of the present application. It should be noted that those of ordinary skill in the art can also make several improvements and modifications without departing from the principle of the present application, and these improvements and modifications should also be considered as the scope of protection of the present application.

## Claims

1. A breast pump, comprising:
a milk storage container, wherein a storage space for storing milk is formed in the milk storage container;
a breast shield, wherein the breast shield is detachably connected to the milk storage container, and a milk expression channel is formed in the breast shield;
a main unit, wherein the main unit is detachably connected to the milk storage container and configured to generate a negative pressure for milk expression in the milk expression channel to drive the milk in the milk expression channel to flow into the storage space; and
a disassembly restricting member, wherein the disassembly restricting member is connected to the main unit, the breast shield, or the milk storage container, and is configured to restrict disassembly between the breast shield and the milk storage container in a state where the main unit is connected to the milk storage container, and allow the disassembly between the breast shield and the milk storage container in a state where the main unit is disconnected from the milk storage container.

2. The breast pump according to claim 1, wherein the disassembly restricting member is connected to the breast shield and is capable of extending between the milk storage container and the main unit, and the disassembly restricting member is clamped between the main unit and the milk storage container in the state where the main unit is connected to the milk storage container.

3. The breast pump according to claim 2, wherein the disassembly restricting member is made of a soft material.

4. The breast pump according to claim 2, wherein the breast shield is at least partially made of a soft material.

5. The breast pump according to claim 2, wherein the disassembly restricting member and the breast shield are integrally formed.

6. The breast pump according to claim 2, wherein the milk storage container and the main unit define an accommodating space, and the disassembly restricting member is accommodated in the accommodating space in the state where the main unit is connected to the milk storage container.

7. The breast pump according to claim 6, wherein a size of a lower portion of the accommodating space is greater than a size of an upper portion of the accommodating space, and the disassembly restricting member matches the accommodating space in shape.

8. The breast pump according to claim 6, wherein the accommodating space comprises an accommodating groove formed in a surface of the milk storage container close to the main unit.

9. The breast pump according to claim 1, wherein the milk storage container and the breast shield define the storage space.

10. The breast pump according to any one of claims 1 to 9, wherein the milk storage container is provided with a milk pouring spout, and the disassembly restricting member is further configured to cover and seal the milk pouring spout.

11. An assembly method for a breast pump, comprising:
providing a main unit, a breast shield, a milk storage container, and a disassembly restricting member;
connecting the breast shield to the milk storage container, and placing the disassembly restricting member at a preset position; and
connecting the milk storage container to the main unit, such that the disassembly restricting member restricts disassembly between the breast shield and the milk storage container.

12. The assembly method according to claim 11, wherein the disassembly restricting member is connected to the breast shield;
the connecting the breast shield to the milk storage container, and placing the disassembly restricting member at a preset position comprises:
connecting the breast shield to the milk storage container, and placing the disassembly restricting member between the milk storage container and the main unit; and
the connecting the milk storage container to the main unit, such that the disassembly restricting member restricts disassembly between the breast shield and the milk storage container comprises:
connecting the milk storage container to the main unit, such that the disassembly restricting member is clamped between the main unit and the milk storage container.

13. A disassembly method for a breast pump, comprising:
disassembling a milk storage container and a main unit to release restriction of a disassembly restricting member on disassembly between the milk storage container and a breast shield; and
disassembling the milk storage container and the breast shield.
